# EUROPEAN PATENT SPECIFICATION

(11) **EP 4 753 576 B1**
(45) Date of publication and mention of the grant of the patent: **25.11.2026**
(21) Application number: 24745775.7
(22) Date of filing: 26.07.2024
(51) Int. Cl.: A61B 8/00, A61B 8/08, G01S 15/89

(54) **LUNG ULTRASOUND IMAGING**
A-LINE-DETEKTION IM LUNGENULTRASCHALL
DÉTECTION DE LIGNE A DANS LES ULTRASONS PULMONAIRES

(30) Priority: 01.08.2023 US 202363530095 P; 31.08.2023 EP 23194515
(43) Date of publication of application: 10.06.2026
(73) Proprietor: Koninklijke Philips N.V., 5656 AE Eindhoven (NL)
(72) Inventor: ZAHIRI, Mohsen, 5656 AG Eindhoven (NL); CHEN, Alvin, 5656 AG Eindhoven (NL); GHOSHAL, Goutam, 5656 AG Eindhoven (NL); BHARAT, Shyam, 5656 AG Eindhoven (NL); ODUNGATTU THODIYIL, Anumod, 5656 AG Eindhoven (NL); SRINIVASA NAIDU, Raghavendra, 5656 AG Eindhoven (NL); RAJU, Balasundar Iyyavu, 5656 AG Eindhoven (NL)
(74) Representative: Philips Intellectual Property & Standards
(86) International application number: PCT/EP2024/071345
(87) International publication number: WO 2025/026936

(56) References cited:
- US-A1- 2022 386 998
- THIERY ORIANE ET AL: "Semi-Automatic A-Line Detection and Confidence Scoring in Lung Ultrasound", 2022 IEEE BIOMEDICAL CIRCUITS AND SYSTEMS CONFERENCE (BIOCAS), IEEE, 13 October 2022 (2022-10-13), pages 635 - 639, XP034228864, DOI: 10.1109/BIOCAS54905.2022.9948591

## Description

### FIELD OF THE INVENTION

The invention relates to medical imaging. In particular to lung ultrasound imaging.

### BACKGROUND OF THE INVENTION

Ultrasound imaging of lungs is a technique deployed at the point-of-care to aid in evaluation of lung pathologies such as pulmonary edema and respiratory infection. Healthy, air-filled lungs are often seen under ultrasound as being filled with A-lines. A-lines are repeating, hyperechoic, horizontal artefacts arising at regular intervals from the pleural line equaling the distance between skin and the pleural line. Specifically, A-lines appear when the ultrasound beam hits the highly reflective pleural surface and is reflected back to the transducer. The first return results in a true image of the pleural surface on the monitor. The beam however reflects back again off the transducer face and the cycle repeats. Reverberation may be defined as a prolongation or continuing effect of a sound after the sound has stopped propagating. Multiple reverberations result in multiple A-lines, at multiples of the pleural depth. In contrast, lungs filled with fluid, as is often the case in patients with heart failure or pneumonia, are typically characterized by B-lines. B-lines appear as dynamic structures extending vertically from the pleural line to the bottom of the screen. B-lines are pathological features that arise as "comet-tail" artefacts due to fluid accumulation in the interstitial space.

Accurately identifying A-lines and B-lines, and discerning A-lines and B-lines from each other and from other lung ultrasound structures of similar appearance is challenging for existing quantitative B-line detection algorithms. Accurately identifying A-lines and B-lines and differentiating A-lines and B-lines from other lung ultrasound structures can be challenging due to similarities in their visual appearance. The effectiveness of lung ultrasound imaging may depend on operator experience, image quality, and selection of imaging settings. For ultrasound users, the evaluation of B-lines can play an important role in screening, diagnosis, or management of disease progression. However, standardization of measurements is not provided for even experienced ultrasound users.

Thierry Oriane et al. 2022 "Semi-automatic A-Line Detection and Confidence Scoring in Lung Ultrasound" discloses a method for A-line detection.

### SUMMARY OF THE INVENTION

It is inter alia an object of the invention to assist in the identification of A-lines in ultrasound imaging.

The invention is defined by the independent claims. The dependent claims define advantageous embodiments.

According to an embodiment, there is provided a computer-implemented method for ultrasound imaging, the method comprising:
receiving an ultrasound image using a transducer array of an ultrasound probe in an ultrasound procedure;
selecting candidate regions of interest in the ultrasound image,
   wherein each candidate region of interest comprises an essentially rectangular region,
   wherein a major axis of each essentially rectangular region is essentially perpendicular to potential A-lines and/or parallel to potential B-lines and wherein a minor axis of each rectangular region is essentially parallel to potential A-lines and/or perpendicular to potential B-lines,
   wherein the essentially rectangular regions are essentially non-overlapping with each other, and
   wherein the essentially rectangular regions are essentially parallel to each other; and
generating, for each candidate region of interest, a reverberation feature score,
generating, based on the reverberation feature score for each candidate region, an aggregate score for reverberations in the ultrasound image;
estimating, based on the reverberation feature score and/or the aggregate score for reverberations, presence of one or more A-line structures in the ultrasound image; and
estimating, based on the reverberation feature score and/or the aggregate score for reverberations, strength of one or more A-line structures in the ultrasound image.

In this way, an ultrasound image is searched for candidate structures column-wise (i.e., along each column), owing to the rectangular search or evaluation windows with the major axis of the rectangular region being vertical, followed by a row-wise aggregation of scores in each search window. Thereby providing a 2-step simple and cheap algorithm that can be implemented in near-real-time owing to its simplicity and low computational cost.

In an example, the method further comprises:
classifying one or more B-line structures in the ultrasound image based on the reverberation feature score and/or the aggregate score for reverberations.

In an example, the method further comprises:
generating pixel data for the ultrasound image; and
determining a pixel intensity for the pixel data; and
wherein the reverberation feature score for each candidate region of interest is based on the pixel intensity within said candidate region of interest.

In an example, selecting candidate regions of interest in the ultrasound image comprises:
identifying, based on the pixel intensity in the ultrasound image, one or more B-line candidates in the ultrasound image; and
defining a candidate region of interest enclosing each identified B-line candidate.

In an example, generating the reverberation feature score for each candidate region of interest based on the pixel intensity within said candidate region of interest comprises for each region of interest:
averaging the pixel intensity data;
fitting the averaged pixel intensity data;
comparing the averaged pixel intensity data to the fitted averaged pixel intensity data; and
identifying one or more maximum absolute differences between the averaged pixel intensity data and the fitted averaged pixel intensity data.

In an example, the method further comprises:
identifying a pleural line in the ultrasound image; and
wherein the candidate regions of interest in the ultrasound image extend from the pleural line.

In an example, the method further comprises:
generating display data for the ultrasound image and markers for at least one of A-line structures or B-line structures.

In an example, the receiving step comprises receiving a plurality of ultrasound images forming a cineloop, and the method further comprises generating a second aggregate score for reverberations, wherein the second aggregate score is based on the reverberation feature score and the aggregate score for reverberation from each of a subset of the ultrasound images in the cineloop.

According to an embodiment, there is provided an ultrasound system, comprising:
a processor configured to carry out any of the methods as disclosed herein.

In an example, the system further comprises:
a display in communication with the processor, wherein the display is configured to display the ultrasound image and markers for at least one of A-line structures or B-line structures.

In an example, the system further comprises:
an ultrasound transducer array in communication with the processor and configured to acquire the ultrasound image.

According to an embodiment, there is provided a computer program product comprising instructions that when executed by a processor cause the processor to carry out any of the methods as disclosed herein. In this disclosure, the notions "computer program", "software program", and "program" have the same meaning.

These and other aspects of the invention will be apparent from and elucidated with reference to the embodiments described hereinafter.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 illustrates an exemplary ultrasound system, in accordance with a representative embodiment.
Fig. 2 illustrates another exemplary ultrasound, in accordance with a representative embodiment.
Fig. 3A illustrates an exemplary method for A-line detection in lung ultrasound, in accordance with a representative embodiment.
Fig. 3B illustrates another exemplary method for A-line detection in lung ultrasound, in accordance with a representative embodiment.
Fig. 4 illustrates examples of A-lines and B-lines for A-line detection in lung ultrasound, in accordance with a representative embodiment.
Fig. 5 illustrates candidate regions of interest in A-line detection in lung ultrasound, in accordance with a representative embodiment.
Fig. 6 illustrates candidate regions of interest in A-line detection in lung ultrasound, in accordance with a representative embodiment.
Fig. 7 illustrates differences between a candidate profile and two types of fitted data in A-line detection in lung ultrasound, in accordance with a representative embodiment.
Fig. 8 illustrates a reverberation feature score evaluated within a reduced window in A-line detection in lung ultrasound, in accordance with a representative embodiment.
Fig. 9 illustrates cineloop-level assessment in A-line detection in lung ultrasound, in accordance with a representative embodiment.
Fig. 10 illustrates a computer system, on which a method for A-line detection in lung ultrasound is implemented, in accordance with a representative embodiment.

### DETAILED DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the context of the present disclosure, "essentially" refers to a condition where the described attribute or characteristic predominantly aligns with the stated quality, form, or orientation, without necessitating absolute precision. For instance, "essentially rectangular" signifies a shape predominantly resembling a rectangle, although minor deviations may be present. Similarly, "essentially parallel" denotes elements or components that are primarily aligned in a parallel manner, allowing for slight variations. "Essentially perpendicular" indicates an orientation predominantly perpendicular, while permitting minor angular deviations. Moreover, "essentially non-overlapping" implies a configuration where objects or elements primarily avoid overlap, acknowledging potential marginal intersections that do not significantly obscure the distinctiveness of the entities involved.

The present disclosure, through one or more of its various aspects, embodiments and/or specific features or sub-components, is thus intended to bring out one or more of the advantages as specifically noted below.

As described herein, A-lines in lung ultrasound video loops may be identified based on reverberation appearance along the ultrasound beam profile. Image metrics describing A-line appearance may be generated to provide cineloop-level assessment of overall lung health. Presence and strength of reverberations may also be used to identify B-lines. Detected A-lines and B-lines may be displayed on an ultrasound image, and cineloop-level results may be summarized across all lung zones via an overall summary screen. Such challenges can potentially be addressed by automated algorithms, particularly in settings where resources and trained professionals are limited. A-lines may be differentiated from B-lines by identifying and characterizing reverberation appearance along the ultrasound beam profile, and this may be used to enhance counting of B-lines. The differentiation may provide an improved ability to identify A-lines as an indicator of healthy, air-filled, lungs.

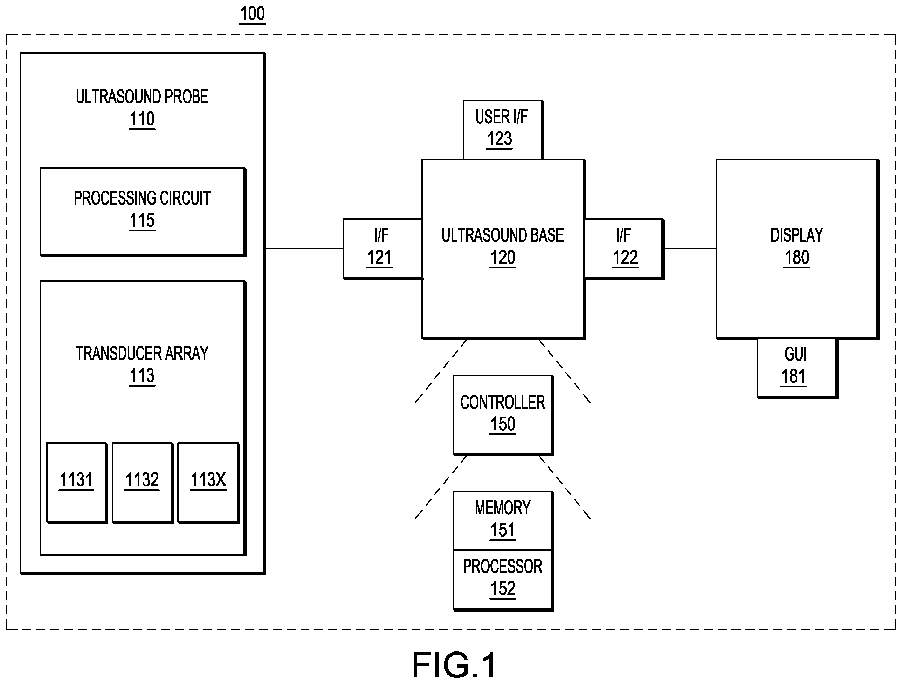

Fig. 1 illustrates a system 100 for A-line detection in lung ultrasound, in accordance with a representative embodiment.

The system 100 in Fig. 1 is a system for A-line detection in lung ultrasound and includes components that may be provided together or that may be distributed. The system 100 includes an ultrasound probe 110, an ultrasound base 120, and a display 180.

The ultrasound probe 110 includes a processing circuit 115 and a transducer array 113. The processing circuit 115 may comprise a memory for storing data and instructions and an application-specific integrated circuit (ASIC) and/or a processor for processing data and instructions. The transducer array 113 includes an array of transducer elements including at least a first transducer element 1131, a second transducer element 1132, and an Xth transducer element 113X. That is, the ultrasound probe 110 comprises an ultrasound transducer and associated electronics to produce images that are then rendered on the display 180 for the user to view. The ultrasound probe 110 includes the transducer array 113. The transducer array 113 converts electrical energy into sound waves which bounce off of body tissue, and receives echoes of the sound waves and converts the echoes into electrical energy. The transducer array 113 may include dozens, hundreds or thousands of individual transducer elements. The ultrasound probe 110 may transmit a beam to produce images and may detect the echoes. The processing circuit 115 may process ultrasound images captured by the transducer array 113 of the ultrasound probe 110.

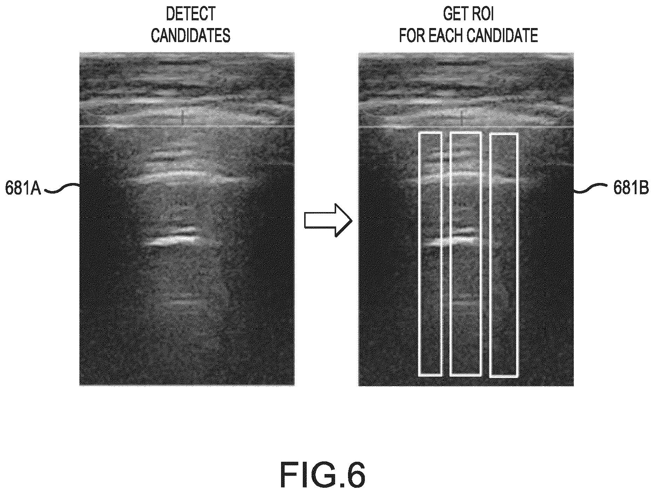

The ultrasound base 120 may comprise or be part of an ultrasound cart. The ultrasound base 120 includes a first interface 121, a second interface 122, a third interface 123, and a controller 150. A computer that can be used to implement the ultrasound base 120 is depicted in Fig. 6, though an ultrasound base 120 may include more or fewer elements than depicted in Fig. 1 or Fig. 6. One or more of the interfaces may include ports, disk drives, wireless antennas, or other types of receiver circuitry that connect the controller 150 to other electronic elements. The first interface 121 connects the ultrasound base 120 to the ultrasound probe 110, and may comprise a port, an antenna, and/or another type of physical component for wired or wireless communications. The second interface 1220 connects the ultrasound base 120 to the display 180, and may also comprise a port, an antenna, and/or another type of physical component for wired or wireless communications. The third interface 123 is a user interface, and may comprise buttons, keys, a mouse, a microphone, a speaker, switches, a touchscreen or other type of display separate from the display 180, and/or other types of physical components that allow a user to interact with the ultrasound base 120 such as to enter instructions and receive output.

The controller 150 includes at least a memory 151 that stores instructions and a processor 152 that executes the instructions. The instructions stored in the memory 151 may comprise a software program for generating a score for estimating presence and strength of one or more A-line structure(s) in an ultrasound image by generating a reverberation feature score in the ultrasound image. The reverberation feature score and markers or other types of labels for A-line structures as well as B-line structures may be generated as display data and displayed on the display 180 overlapped on or provided adjacent to ultrasound images. The software program in the memory 151 may help ensure appropriate ultrasound images are obtained during an ultrasound examination before a patient leaves.

The display 180 may be local to the ultrasound base 120 or may be remotely connected to the ultrasound base 120, such as wirelessly. The display 180 may be connected to the ultrasound base 120 via a local wired interface such as an Ethernet cable or via a local wireless interface such as a Wi-Fi connection. The display 180 may be interfaced with other user input devices by which users can input instructions, including mouses, keyboards, thumbwheels and so on. The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect to other elements or components, as well as an interactive touch screen configured to display prompts to users and collect touch input from users. The display is configured to display markers for at least one of A-line structures and B-line structures in ultrasound images.

The controller 150 may perform some of the operations described herein directly and may implement other operations described herein indirectly. For example, the controller 150 may indirectly control operations such as by generating and transmitting content to be displayed on the display 180. The controller 150 may directly control other operations such as logical operations performed by the processor 152 executing instructions from the memory 151 based on input received from electronic elements and/or users via the interfaces. Accordingly, the processes implemented by the controller 150 when the processor 152 executes instructions from the memory 151 may include steps not directly performed by the controller 150.

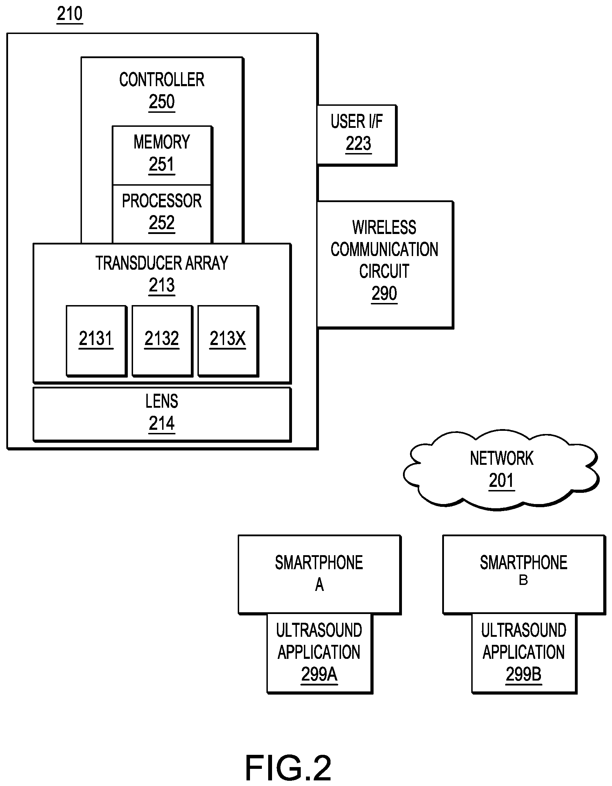

Fig. 2 illustrates another system for A-line detection in lung ultrasound, in accordance with a representative embodiment.

In Fig. 2, an ultrasound probe 210 and a smartphone A and a smartphone B are connected over a network 201.

The network 201 may comprise a local wireless network such as a WiFi network, though the network 201 may also or alternatively include wired elements such as wires connected to smartphone A and smartphone B via USB cables.

The ultrasound probe 210 includes a transducer array 213, a lens 214, a user interface 223, a controller 250 and a wireless communication circuit 290. The transducer array 213 includes at least a first transducer element 2131, a second transducer element 2132, and an Xth transducer element 213X. The transducer array 213 may correspond to the transducer array 113 and may comprise an array of individually controllable transducer elements. The transducer array 213 converts electrical energy into sound waves which bounce off of body tissue, and receives echoes of the sound waves and converts the echoes into electrical energy. The transducer array 213 may include dozens, hundreds or thousands of individual transducer elements. The ultrasound probe 210 may transmit a beam to produce images and may detect the echoes. The lens 214 may be used to transmit ultrasound beams and to receive echoes of ultrasound beams. The user interface 223 may be used by a user to interact with the ultrasound probe 210. The wireless communication circuit 290 may be used to communicate with smartphone A and smartphone B via the network 201. That is, the ultrasound probe 210 comprises an ultrasound transducer and associated electronics to produce images that are then rendered on displays of smartphone A and/or smartphone B.

Smartphone A stores and executes an ultrasound application 299A. Smartphone B stores and executes an ultrasound application 299B. The ultrasound application 299A and the ultrasound application 299B may be configured to enable smartphone A and smartphone B interact with the ultrasound probe 210 via the network 201. For example, ultrasound application 299A and ultrasound application 299B may be configured to enable displays of ultrasound images from the ultrasound probe 210. Ultrasound application 299A and ultrasound application 299B may also be configured to overlay scores and labels and/or other markers on the displays of ultrasound images from the ultrasound probe 210. The ultrasound probe 210 is configured to link to each of smartphone A and/or smartphone B as an external device for the ultrasound procedure via the ultrasound application 299A and/or ultrasound application 299B. The ultrasound image may be displayed on smartphone A and/or smartphone B as the external device, and the external device is configured to display markers for at least one of A-line structures or B-line structures in the ultrasound image.

The controller 250 includes at least a memory 251 that stores instructions and a processor 252 that executes the instructions. The instructions stored in the memory 251 may comprise a software program with a model such as an artificial intelligence model, as well as the same or a different software program for generating a user interface on an ultrasound device. In Fig. 2, the user interface may be generated by the instructions stored in the memory 251 and may be displayed on a display of the smartphone A or smartphone B. The software program(s) in the memory 251 generate scores and labels and/or other markers for A-lines as well as B-lines when present in an ultrasound image.

The controller 250 may perform some of the operations described herein directly and may implement other operations described herein indirectly. For example, the controller 250 may indirectly control operations such as by generating and transmitting content to be displayed on a display of the smartphone A or smartphone B. The controller 250 may directly control other operations such as logical operations performed by the processor 252 executing instructions from the memory 251 based on input received from electronic elements and/or users via the interfaces. Accordingly, the processes implemented by the controller 250 when the processor 252 executes instructions from the memory 251 may include steps not directly performed by the controller 250.

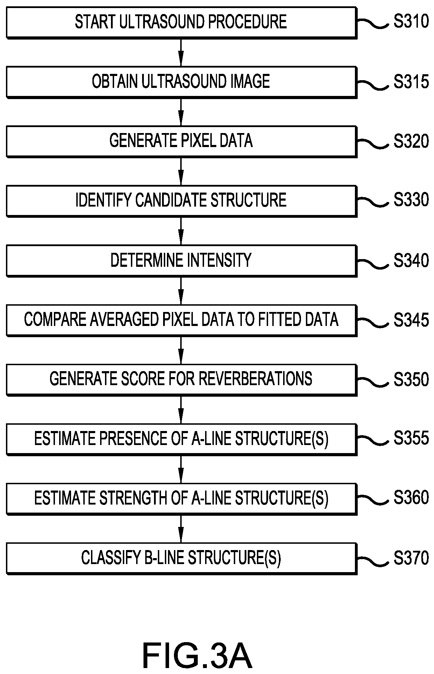

Fig. 3A illustrates a method for A-line detection in lung ultrasound, in accordance with a representative embodiment.

The method of Fig. 3A may be performed by the system 100 including the controller of the ultrasound base 120 in Fig. 1, or by the ultrasound probe 210 in Fig. 2.

At S310, an ultrasound procedure is started. The ultrasound procedure may involve obtaining a cineloop of ultrasound images using an ultrasound probe such as the ultrasound probe 110 or the ultrasound probe 210.

At S315, an ultrasound image is obtained during the ultrasound procedure. The ultrasound image at S315 may comprise a single ultrasound image captured at a point in time, but in the context of a cineloop in which a sequence of two or more ultrasound images are captured. Each ultrasound image is obtained using a transducer array of an ultrasound probe such as the ultrasound probe 110 or the ultrasound probe 210.

At S320, pixel data is generated from the ultrasound images obtained by the transducer array. The ultrasound probe 110 or the ultrasound probe 210 may generate rows and columns of pixel data for each ultrasound image using the transducer array.

At S330, one or more candidate structure(s) are identified. For example, a frame may include multiple candidate structure(s) for A-lines. The candidate structures may be identified by an image analysis program that checks for patterns in the ultrasound images.

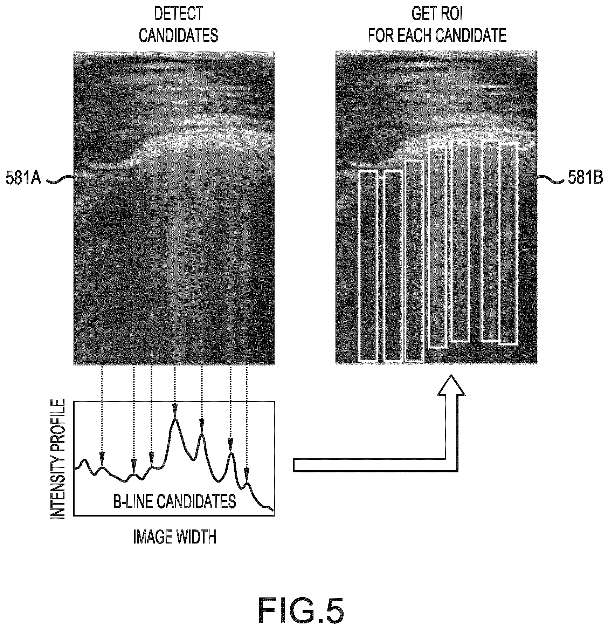

B-line candidate structures are detected as local peaks in the vertical direction, which can be detected from the image intensity profile of the image. The intensity profile is computed along each column of the image to generate a plot such as shown on the bottom left in Fig. 5. Each peak in the plot corresponds to a candidate B-line.

A-line candidate structures are determined in a second step by analyzing the smaller rectangular region-of-interest generated from each B-line candidate (local peak), as seen for example in 681B and described later with respect to Fig. 6. A-lines appear as peaks along the horizontal direction, and can be detected as local peaks in the intensity profiles for each smaller region-of-interest. Here, unlike above, the intensity profile is computed along each row of the region-of-interest. Of note, since A-lines stretch horizontally across the image, the same A-line tends to appear as local peaks across multiple adjacent regions-of-interest.

Alternatively, A-line candidate structures may be detected directly from the image based on the intensity of the pixels, e.g. along each row of pixels of the image, and finding local peaks. This bypasses the need to first detect B-line candidate structures before identifying the A-line candidates.

At S340, an intensity is determined. The ultrasound probe 110, the ultrasound base 120 and/or the ultrasound probe 210 may be configured to determine intensity among rows and columns of pixel data. The intensity is determined for pixel data of each candidate structure identified in a frame at S330, so intensity may be separately and independently determined for pixel data of multiple candidate structures in the current frame at S340. The intensity may be determined among some or all rows and columns of pixel data. In some embodiments, intensity may be determined for fewer than all pixels represented by pixel data in the ultrasound image. For example, for a smaller window within the ultrasound image. In some embodiments, a candidate structure in the ultrasound image may be identified, and the intensity determined at S340 may be limited to intensity for rows and columns of pixel data for the candidate structure or a set of such candidate structures.

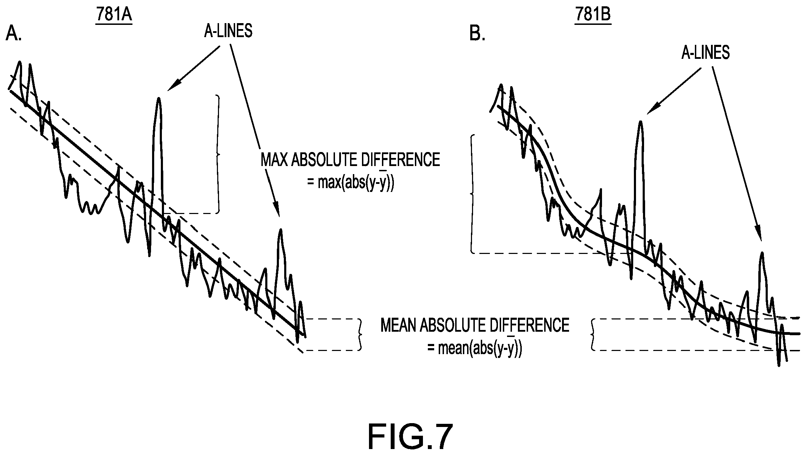

At S345, averaged pixel data is compared to fitted data. The ultrasound base 120 or the ultrasound probe 210 may average pixel data for a candidate structure to generate for example either of the jagged lines in Fig. 7. The average pixel data for all candidate structures may then be fitted to generate for example the smooth continuous lines in Fig. 7. The ultrasound base 120 or the ultrasound probe may then identify one or more maximum absolute differences between averaged pixel data for each candidate structure and the fitted data. In other words, the large deviations between the noisy line and smooth continuous line as shown in Fig. 7 may be identified and classified. The comparison may be between a raw intensity profile and linear or nonlinear best-fit profiles. The comparison at S345 is for pixel data for one candidate structure, and the comparison may be performed separately and independently for the averaged pixel data of each candidate structure in the frame at S345.

At S350, a reverberation feature score is generated. The reverberation feature score may be or may reflect image metrics describing A-line appearance in order to provide an assessment of overall lung health. The reverberation feature score is a score that measures likelihood of A-line presence in the ultrasound image based on the intensity determined at S340.

In some embodiments, the reverberation feature score generated at S350 may measure likelihood of A-line presence, but may also be generated based on detecting an initial set of one or more potential B-line candidates in each ultrasound frame. B-line candidates may be identified as local peaks in the intensity profile along the lateral width of an ultrasound frame. For each detected candidate, a smaller region of interest may be defined, and the reverberation feature score may be computed from the regions of interest. The concept of identifying B-line candidates to score the likelihood of A-line presence is shown in and described with respect to Fig. 5 below. In contrast, Fig. 6 shows an example of candidate regions of interest computed from an ultrasound image frame containing many A-lines and no B-lines, so that the reverberation feature score at S350 is generated based detecting an initial set of A-line candidates.

At S355, presence of A-line structures is estimated. Presence of A-line structures may be estimated based on the comparison at S345 and the reverberation feature score generated at S350.

At S360, strength of A-line structures is estimated. Strength of A-line structures may be estimated based on the reverberation feature score, and may be analogous to a confidence score indicating the confidence of a candidate being an A-line structure. For example, strength of an A-line structure may be given a score from 0 to 100 so as to provide strong granularity.

At S370, B-line structures are classified. B-line structures in each frame of a cineloop may be classified using the reverberation feature score. A candidate structure may be classified as a B-line structure based on the intensity determined for the rows and columns of pixel data for a candidate structure. In some embodiments, the reverberation feature score may be used in combination with other image features to classify the B-lines in each frame. Each identified A-line and/or B-line in a frame may be displayed on the display 170, the ultrasound probe 210 or one of smartphone A or smartphone B in each frame, such as in each frame of a cineloop. Detected A-lined and B-lines may be displayed in the form of bounding boxes, for example.

The method of Fig. 3A identifies A-lines in ultrasound images of a lung ultrasound video loop, and differentiates the A-lines from B-lines, based on reverberation appearance along the ultrasound beam profile.

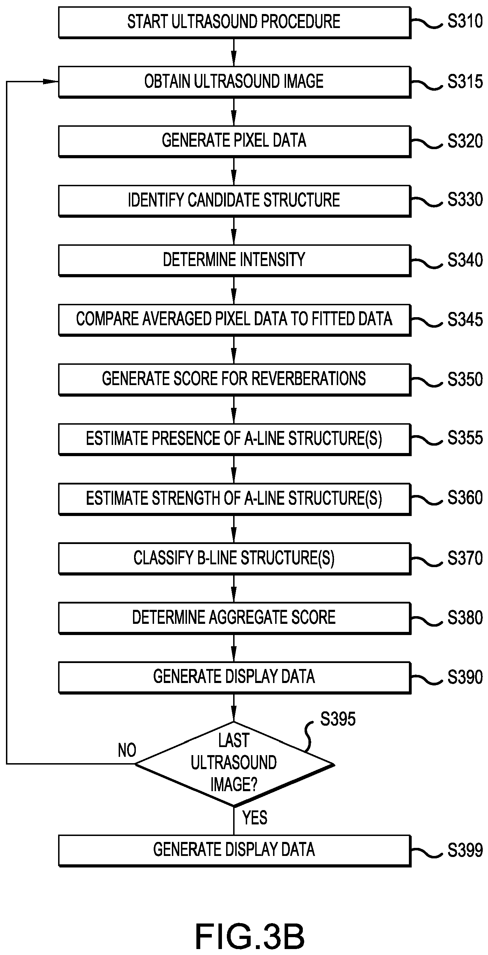

Fig. 3B illustrates another method for A-line detection in lung ultrasound, in accordance with a representative embodiment.

The method of Fig. 3B may be performed by the system 100 including the controller 150 of the ultrasound base 120 in Fig. 1, or by the ultrasound probe 210 in Fig. 2.

The method of Fig. 3B largely overlaps with the method of Fig. 3A, except that the method is performed for a plurality of ultrasound images in a cineloop. At S310, an ultrasound procedure is started. At S315, an ultrasound image is obtained during the ultrasound procedure. At S320, pixel data is generated from the ultrasound images. At S330, one or more candidate structure(s) are identified. At S340, intensity is determined. At S345, averaged pixel data is compared to fitted data. At S350, a reverberation feature score is generated. The reverberation feature score may be or may reflect image metrics describing A-line appearance in order to provide an assessment of overall lung health, and may be used to provide a cineloop-level assessment in the context of Fig. 3B. The method of Fig. 3B identifies A-lines in ultrasound images of a lung ultrasound video loop, and differentiates the A-lines from B-lines, based on reverberation appearance along the ultrasound beam profile.

At S355, presence of A-line structures is estimated. At S360, strength of A-line structures is estimated. At S370, B-line structures are classified. Each identified A-line and/or B-line in a frame may be displayed on the display 170, the ultrasound probe 210 or one of smartphone A or smartphone B in each frame, such as in each frame of a cineloop. Detected A-lined and B-lines may be displayed in the form of bounding boxes, for example.

At S380, an aggregate score is determined. The aggregate score may be determined at S380 after each ultrasound frame scored for reverberations at S350, and may be considered an update of a previous aggregate score when determined a second or subsequent time for a cineloop in Fig. 3B. A cineloop-level assessment of presence of A-lines can also be provided to the user as an overall measure of lung health. The aggregate score from S380 may be an overall score for an entire cineloop or may be for each frame or subsets of the frames in a cineloop.

At S390, display data is generated. The display data generated at S390 may be display data for the aggregate score determined at S380. The display data may be generated by the controller 150 in Fig. 1, and then provided to the display 180. The display data may be generated by the controller 250 in Fig. 2, and then provided to a display of the ultrasound probe 210, or to smartphone A or smartphone B for display.

At S395, a determination is made as to whether the ultrasound image obtained at S315 is the last ultrasound image. If the ultrasound image obtained at S315 is not the last ultrasound image (S395 = No), the method returns to S315 and another ultrasound image is obtained.

If the ultrasound image obtained at S315 is the last ultrasound image (S395 = Yes), at S399 display data is again generated. Display data generated at S395 may be for an aggregate score for A-line structures identified in a cineloop. The quantification evident in the methods of Fig. 3A and Fig. 3B may be used as inputs for prediction of whether a lung being imaged is normal or abnormal, and such a prediction may also be output as display data at S399. In some embodiments, such as with portable ultrasound systems which may be used by end users, such predictions may be limited and may instead be made by a system controlled by a health care provider.

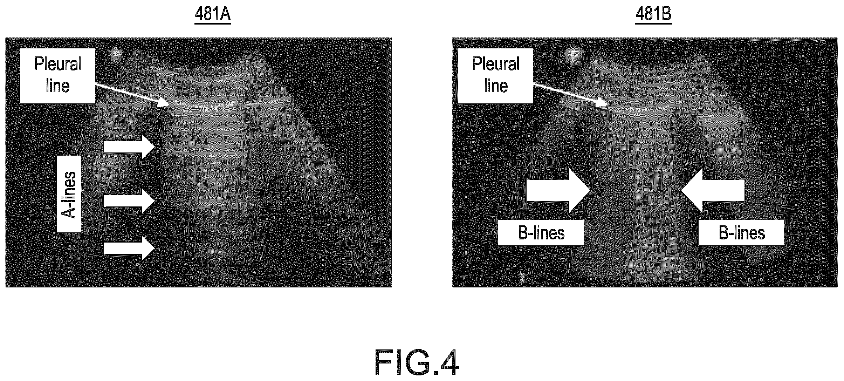

Fig. 4 illustrates examples of A-lines and B-lines for A-line detection in lung ultrasound, in accordance with a representative embodiment.

In Fig. 4, examples of A-lines are shown on the left on a first user interface 481A, and B-lines are shown on the right on a second user interface 481B. The A-lines and B-lines in Fig. 4 are in lung ultrasound images. A-lines are a sign of healthy air-filled lung, whereas B-lines are a pathological sign of lung fluid.

Fig. 5 illustrates candidate regions of interest in A-line detection in lung ultrasound, in accordance with a representative embodiment.

As noted above with respect to S350, a score may measure likelihood of A-line presence, but may be generated based on detecting an initial set of one or more potential B-line candidates in each ultrasound frame. In Fig. 5, pixel data in an ultrasound frame shown in user interface 581A on the left is analyzed to identify B-line candidates as local peaks in the intensity profile computed along the lateral width of an ultrasound frame. For each detected candidate, a smaller region of interest may be defined as in user interface 581B, and the reverberation feature score may be computed from the regions of interest. Example candidate regions of interest shown on user interface 581B are computed from an ultrasound image frame containing many B-lines shown in the user interface 581A. Each of the B-line candidates have a fairly uniform intensity extending vertically from the pleural line to the bottom of the image.

Fig. 6 illustrates candidate regions of interest in A-line detection in lung ultrasound, in accordance with a representative embodiment.

In Fig. 6, candidate regions of interest are computed from an ultrasound image frame containing many A-lines and no B-lines, so that the reverberation feature score at S350 is generated based on detecting an initial set of A-line candidates. In Fig. 6, the candidates are shown on the user interface 681A on the left, and then a region of interest is obtained for each candidate in the user interface 681B on the right. The candidate regions of interest in Fig. 6 are computed from an ultrasound image frame containing many A-lines and no B-lines. Distinct spikes are shown for local peaks along the intensity profiles with each candidate region of interest in Fig. 6.

As an example, the bounding boxes in Fig. 6 may comprise rectangles with widths of 10 pixels and heights of 200 pixels. Intensity may be provided as a mean of a row, such as 10 pixels for each point of the height. 10 x 200 pixels as dimensions for a bounding box is only an example, and candidate bounding boxes may be smaller or larger in either or both dimensions. In other examples the height to width ratio may be 2, 4, 6, 8, 10, 12, 14, 16 or 18. In other words, for example 2 pixel width and 4 pixels height, 20 pixel width and 40 pixel height, 10 pixel width and 100 pixel height, etc. The rectangles 681B as shown in Fig. 6 may comprise a major axis in the vertical direction and a minor axis in the horizontal direction.

Fig. 7 illustrates differences between a candidate profile and two types of fitted data in A-line detection in lung ultrasound, in accordance with a representative embodiment.

As explained above, the reverberation appearance of each candidate may be computed inside a corresponding candidate region of interest. Specifically, for each candidate, a reverberation feature score is calculated as the mean absolute difference between the raw intensity profile along the center of the region of interest compared to the linear best-fit of that profile. If y is considered the intensity profile along the centerline of the candidate region of interest and y' is the linear best fit of the intensity profile the reverberation feature score may be calculated as the mean of the absolute different difference between the intensity profile along the centerline and the linear best fit of the intensity profile along the centerline. The user interface 781A on the left in Fig. 7 shows a visual representation of the reverberation feature calculated based on linear best-fit. The user interface 781B on the right in Fig. 7 shows a visual representation of the reverberation feature calculated based on a smoothed nonlinear best-fit, which could be described as a(y) rather than y' for a linear best fit, wherein "a" represents a smoothing operator such as Gaussian smoothing, median smoothing, frequency/Fourier-based smoothing or any other denoising filter. The smoothing operator "a" may be applied to the original intensity profile y directly or may be applied to the intensity profile y after pre-processing of the intensity profile signal by a separate operator. In both user interfaces in Fig. 7, the jagged line with peaks referenced as "A-lines" is the spot readings of intensity from top to bottom. The center line among the three parallel lines in both user interfaces is the best fit, so linear on the user interface 781A and smoothed nonlinear on the user interface 781B. The upper and lower of the three parallel lines in both user interfaces show the confidence interval, though a confidence interval is not particularly necessary to show the linear best-fit and the smoothed nonlinear best-fit.

A reduced evaluation window may be used instead of the entire length of the profile. The ultrasound probe 110, the ultrasound base 120 and/or the ultrasound probe 210 may be configured to select an area, e.g., of rows and columns of pixels, in the ultrasound image as a window, and generate a reverberation feature score in the window. The reverberation feature score may then be calculated on only a portion of the profile. For example, the middle 50% of the profile may be used for an evaluation window. Additionally, the reverberation feature score may comprise a weighted sum of sub-scores from different evaluation sub-windows. The evaluation inside a smaller window may be used to avoid areas along the candidate profile that are likely to contain noisy peaks due to features other than A-lines, such as portions of the pleural line that may appear near the upper part of the region of interest, or organ structures that may appear near the bottom of the region of interest. Other types of fits, metrics, windows and sub-window sizes can be used to calculate the reverberation feature score that reflects the likelihood of A-line presence, so that such features are not limited to the particular examples set forth herein.

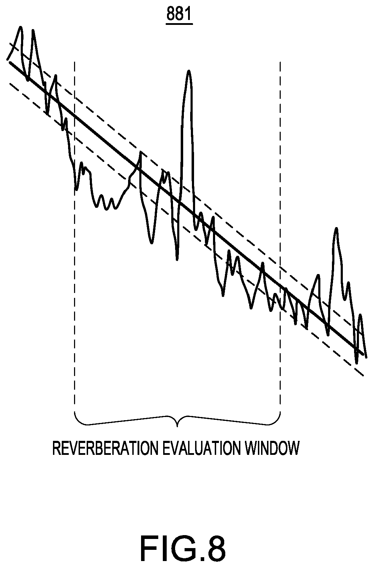

Fig. 8 illustrates a reverberation feature score evaluated within a reduced window in A-line detection in lung ultrasound, in accordance with a representative embodiment.

A visual example of a smaller evaluation window is shown on the user interface 881 in Fig. 8. The reduced window on the user interface 881 is between the dotted vertical lines. For example, instead of using 200 pixels as the width of the window, the smaller evaluation window may include 40 pixels in which intensity changes the most.

In other embodiments, the reverberation feature score may be calculated based on alternative metrics, such as maximum absolute difference, median absolute difference, sum of absolute differences, or combinations of these alternative metrics.

Similarly, squared differences in intensity profile may be used in place of absolute difference, and may emphasize large local peaks corresponding to large differences. Maximum/median/sum of squared difference, or combinations of these alternative metrics may also be used.

Statistical measures may also be used instead of the best-fit profile. Statistical measures include, for example, standard deviation of the intensity profile, or variance of the intensity profile. These types of statistical measures may be used to avoid computing a best-fit line or best-fit curve, so as to simplify processing.

A fast Fourier transformation (FFT) profile may also be used insofar as the shape patterns in the reverberation profile may be evaluated in the frequency domain. For example, a fast Fourier transform of the profiles shown in Fig. 7 may be used to show the frequency spectrum of the profile. A-line peaks that repeat at a uniform increment may be clearly seen in the frequency spectrum from the fast Fourier transform, and may be extracted as a metric.

It is also possible that the exact form of the nonlinear best-fit a(y), and/or the metric for calculating the reverberation feature score, is learned with a data-driven approach using machine learning techniques. Here, rather than pre-defining the parameters of the fitting function "a" and/or the metric, these are determined empirically using a training dataset.

In some embodiments, presence of A-lines may be detected in the image frame since an ultrasound frame may contain multiple candidates, and an A-line may span across multiple candidate regions. Reverberation feature scores for each candidate may be combined to generate a single A-line score for the frame, so as to calculate the average reverberation feature score across all candidates. In alternative embodiments, the reverberation feature score may be calculated as the sum of the reverberation feature score across all candidates. The reverberation feature score may indicate the likelihood of an A-line being present in the frame.

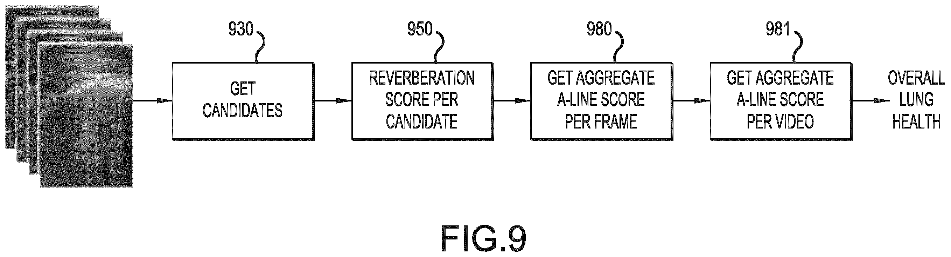

Fig. 9 illustrates cineloop-level assessment in A-line detection in lung ultrasound, in accordance with a representative embodiment.

The cineloop-level assessment of presence of A-lines in Fig. 9 is based on aggregation of individual candidates across the frame and finally over the entire cineloop. The cineloop-level result is provided to the user as an overall measure of lung health.

At 930, candidates are obtained. At 950, a reverberation feature score is calculated per candidate in a frame. At 980, an aggregate score is obtained for the frame. At 981, an aggregate score is obtained for the video. The result of the aggregate score for the video may be output as an indication of overall lung health. A-line scores for each frame may be added together to produce an overall score for the cineloop, and the video-level score may then be used to evaluate the likelihood that the video contains A-lines, as well as the number of A-lines. Presence and quantity of A-lines is indicative of the healthiness (i.e., lack of fluid) in a lung zone. As such, the reverberation feature score may be useful in assessing the overall health of the lung.

In some embodiments, the reverberation feature score may be used in combination with other image features to identify B-lines. That is, in addition to using the reverberation feature score to detect A-lines, the reverberation feature score may also be used to detect B-lines. Unlike A-lines, which are a sign of healthy, air-filled, lungs, B-lines are a pathological sign of lung fluid. Since B-lines tend to appear in the absence of A-lines, a high reverberation feature score (which would suggest higher likelihood of an A-line) would indicate lower likelihood of B-lines and therefore lung fluid. A lower reverberation score (which would suggest higher likelihood of B-lines) would indicate lower likelihood of A-lines.

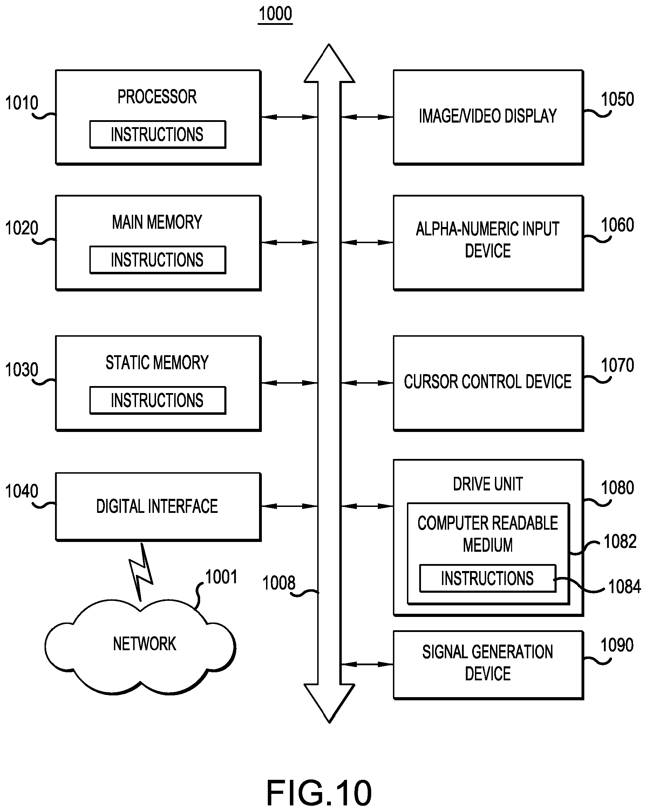

Fig. 10 illustrates a computer system, on which a method for A-line detection in lung ultrasound is implemented, in accordance with another representative embodiment.

Referring to Fig. 4, the computer system 1000 includes a set of software instructions that can be executed to cause the computer system 1000 to perform any of the methods or computer-based functions disclosed herein. The computer system 1000 may operate as a standalone device or may be connected, for example, using a network 1001, to other computer systems or peripheral devices. In embodiments, a computer system 1000 performs logical processing based on digital signals received via an analog-to-digital converter.

In a networked deployment, the computer system 1000 operates in the capacity of a server or as a client user computer in a server-client user network environment, or as a peer computer system in a peer-to-peer (or distributed) network environment. The computer system 1000 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 1000 can be incorporated as or in a device that in turn is in an integrated system that includes additional devices. In an embodiment, the computer system 1000 can be implemented using electronic devices that provide voice, video or data communication. Further, while the computer system 1000 is illustrated in the singular, the term "system" shall also be taken to include any collection of systems or sub-systems that individually or jointly execute a set, or multiple sets, of software instructions to perform one or more computer functions.

As illustrated in Fig. 10, the computer system 1000 includes a processor 1010. The processor 1010 may be considered a representative example of a processor of a controller and executes instructions to implement some or all aspects of methods and processes described herein. The processor 1010 is an article of manufacture and/or a machine component. The processor 1010 is configured to execute software instructions to perform functions as described in the various embodiments herein. The processor 1010 may be a general-purpose processor or may be part of an application specific integrated circuit (ASIC). The processor 1010 may also be a microprocessor, a microcomputer, a processor chip, a controller, a microcontroller, a digital signal processor (DSP), a state machine, or a programmable logic device. The processor 1010 may also be a logical circuit, including a programmable gate array (PGA), such as a field programmable gate array (FPGA), or another type of circuit that includes discrete gate and/or transistor logic. The processor 1010 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 1000 further includes a main memory 1020 and a static memory 1030, where memories in the computer system 1000 communicate with each other and the processor 1010 via a bus 1008. Either or both of the main memory 1020 and the static memory 1030 may be considered representative examples of a memory of a controller, and store instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 1020 and the static memory 1030 are articles of manufacture and/or machine components. The main memory 1020 and the static memory 1030 are computer-readable mediums from which data and executable software instructions can be read by a computer (e.g., the processor 1010). Each of the main memory 1020 and the static memory 1030 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 1000 further includes a video display unit 1050, such as a liquid crystal display (LCD), an organic light emitting diode (OLED), a flat panel display, a solid-state display, or a cathode ray tube (CRT), for example. Additionally, the computer system 1000 includes an input device 1060, such as a keyboard/virtual keyboard or touch-sensitive input screen or speech input with speech recognition, and a cursor control device 1070, such as a mouse or touch-sensitive input screen or pad. The computer system 1000 also optionally includes a disk drive unit 1080, a signal generation device 1090, such as a speaker or remote control, and/or a network interface device 1040.

In an embodiment, as depicted in Fig. 10, the disk drive unit 1080 includes a computer-readable medium 1082 in which one or more sets of software instructions 1084 (software) are embedded. The sets of software instructions 1084 are read from the computer-readable medium 1082 to be executed by the processor 1010. Further, the software instructions 1084, when executed by the processor 1010, perform one or more steps of the methods and processes as described herein. In an embodiment, the software instructions 1084 reside all or in part within the main memory 1020, the static memory 1030 and/or the processor 1010 during execution by the computer system 1000. Further, the computer-readable medium 1082 may include software instructions 1084 or receive and execute software instructions 1084 responsive to a propagated signal, so that a device connected to a network 1001 communicates voice, video or data over the network 1001. The software instructions 1084 may be transmitted or received over the network 1001 via the network interface device 1040.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

In an embodiment, the instructions may form part of a computer program product. The computer program product may be software comprising the instructions, available for download from a server, e.g., via the internet. Alternatively, the instructions may be stored on a suitable (non-transitory) computer-readable medium such as an optical storage medium or a solid-state medium, which may or may not be supplied together with or as part of other hardware.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, A-line detection in lung ultrasound enables differentiation between A-lines and B-lines. A-line detection in lung ultrasound is relatively computationally efficient and may be implemented in real-time in a variety of imaging contexts including with varied organ structures with different A-line and B-line appearance.

Although A-line detection in lung ultrasound has been described with reference to several exemplary embodiments, it is understood that the words that have been used are words of description and illustration, rather than words of limitation. Changes may be made within the purview of the appended claims, as presently stated and as amended, without departing from the scope of A-line detection in lung ultrasound in its aspects. Although A-line detection in lung ultrasound has been described with reference to particular means, materials and embodiments, A-line detection in lung ultrasound is not intended to be limited to the particulars disclosed; rather A-line detection in lung ultrasound extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

The reference signs in the claims shall not be construed as limiting the claims. Measures recited in mutually dependent claims may advantageously be used in combination.

## Claims

1. A computer-implemented method for ultrasound imaging, the method comprising:
receiving (S315) an ultrasound image using a transducer array (113) of an ultrasound probe (110) in an ultrasound procedure;
selecting (S330) candidate regions of interest (581B, 681B) in the ultrasound image,
wherein each candidate region of interest comprises an essentially rectangular region,
wherein a major axis of each essentially rectangular region is essentially perpendicular to potential A-lines and/or parallel to potential B-lines and wherein a minor axis of each rectangular region is essentially parallel to potential A-lines and/or perpendicular to potential B-lines,
wherein the essentially rectangular regions are essentially non-overlapping with each other, and
wherein the essentially rectangular regions are essentially parallel to each other; and
generating (S350), for each candidate region of interest, a reverberation feature score,
generating (S350), based on the reverberation feature score for each candidate region, an aggregate score for reverberations in the ultrasound image;
estimating (S355), based on the reverberation feature score and/or the aggregate score for reverberations, presence of one or more A-line structures in the ultrasound image; and
estimating (S360), based on the reverberation feature score and/or the aggregate score for reverberations, strength of one or more A-line structures in the ultrasound image.

2. The method of claim 1, further comprising:
classifying (S370) one or more B-line structures in the ultrasound image based on the reverberation feature score and/or the aggregate score for reverberations.

3. The method of claim 1 or 2, further comprising:
generating (S320) pixel data for the ultrasound image; and
determining (S340) a pixel intensity for the pixel data; and
wherein the reverberation feature score for each candidate region of interest is based on the pixel intensity within said candidate region of interest.

4. The method of claim 3, wherein selecting candidate regions of interest in the ultrasound image comprises:
identifying, based on the pixel intensity in the ultrasound image, one or more B-line candidates in the ultrasound image; and
defining a candidate region of interest enclosing each identified B-line candidate.

5. The method of claim 3 or 4, wherein generating the reverberation feature score for each candidate region of interest based on the pixel intensity within said candidate region of interest comprises for each region of interest:
averaging the pixel intensity data;
fitting the averaged pixel intensity data;
comparing (S345) the averaged pixel intensity data to the fitted averaged pixel intensity data; and
identifying one or more maximum absolute differences between the averaged pixel intensity data and the fitted averaged pixel intensity data.

6. The method of any one of the preceding claims further comprising:
identifying a pleural line in the ultrasound image; and
wherein the candidate regions of interest in the ultrasound image extend from the pleural line.

7. The method of any one of the preceding claims, further comprising:
generating (S390, S399) display data for the ultrasound image and markers for at least one of A-line structures or B-line structures.

8. The method of any one of the preceding claims, wherein the receiving step comprises receiving a plurality of ultrasound images forming a cineloop, and the method further comprises:
generating a second aggregate score for reverberations, wherein the second aggregate score is based on the reverberation feature score and the aggregate score for reverberation from each of a subset of the ultrasound images in the cineloop.

9. An ultrasound system (100), comprising:
a processor (152, 252) configured to carry out the method of any one of claims 1 to 8.

10. The ultrasound system of claim 9, further comprising:
a display (180) in communication with the processor, wherein the display is configured to display the ultrasound image and markers for at least one of A-line structures or B-line structures.

11. The ultrasound system of claim 9 or 10 further comprising:
an ultrasound transducer array (113, 213) in communication with the processor and configured to acquire the ultrasound image.

12. A computer program product comprising instructions that when executed by a processor cause the processor to carry out the method of any one of claims 1 to 8.

## Patentansprüche

1. Computerimplementiertes Verfahren zur Ultraschallbildgebung, wobei das Verfahren umfasst:
Empfangen (S315) eines Ultraschallbilds unter Verwendung eines Wandlerarrays (113) einer Ultraschallsonde (110) in einer Ultraschallprozedur;
Auswählen (S330) möglicher Bereiche von Interesse (581B, 681B) im Ultraschallbild,
wobei jeder mögliche Bereich von Interesse einen im Wesentlichen rechteckigen Bereich umfasst,
wobei eine Hauptachse jedes im Wesentlichen rechteckigen Bereichs im Wesentlichen senkrecht zu potentiellen A-Linien und/oder parallel zu potentiellen B-Linien ist und wobei eine Nebenachse jedes rechteckigen Bereichs im Wesentlichen parallel zu potentiellen A-Linien und/oder senkrecht zu potentiellen B-Linien ist,
wobei die im Wesentlichen rechteckigen Bereiche sich im Wesentlichen nicht überlappen und
wobei die im Wesentlichen rechteckigen Bereiche im Wesentlichen parallel zueinander sind; und
Erzeugen (S350), für jeden möglichen Bereich von Interesse, einer Nachhallmerkmalsbewertung,
Erzeugen (S350), basierend auf der Nachhallmerkmalsbewertung für jeden möglichen Bereich, einer Gesamtbewertung für Nachhalle im Ultraschallbild;
Schätzen (S355), basierend auf der Nachhallmerkmalsbewertung und/oder der Gesamtbewertung für Nachhalle, des Vorhandenseins einer oder mehrerer A-Linien-Struktur(en) im Ultraschallbild und
Schätzen (S360), basierend auf der Nachhallmerkmalsbewertung und/oder der Gesamtbewertung für Nachhalle, der Stärke einer oder mehrerer A-Linien-Struktur(en) im Ultraschallbild.

2. Verfahren nach Anspruch 1, ferner umfassend:
Klassifizieren (S370) einer oder mehrerer B-Linien-Struktur(en) im Ultraschallbild basierend auf der Nachhallmerkmalsbewertung und/oder der Gesamtbewertung für Nachhalle.

3. Verfahren nach Anspruch 1 oder 2, ferner umfassend:
Erzeugen (S320) von Pixeldaten für das Ultraschallbild und
Bestimmen (S340) einer Pixelintensität für die Pixeldaten; und
wobei die Nachhallmerkmalsbewertung für jeden möglichen Bereich von Interesse auf der Pixelintensität innerhalb des möglichen Bereichs von Interesse basiert.

4. Verfahren nach Anspruch 3, wobei das Auswählen von möglichen Bereichen von Interesse im Ultraschallbild umfasst:
Identifizieren, basierend auf der Pixelintensität im Ultraschallbild, eines oder mehrerer B-Linienkandidaten im Ultraschallbild und
Definieren eines möglichen Bereichs von Interesse, der jeden identifizierten B-Linienkandidaten umschließt.

5. Verfahren nach Anspruch 3 oder 4, wobei das Erzeugen der Nachhallmerkmalsbewertung für jeden möglichen Bereich von Interesse basierend auf der Pixelintensität innerhalb des möglichen Bereichs von Interesse für jeden Bereich von Interesse umfasst:
Mitteln der Pixelintensitätsdaten;
Anpassen der gemittelten Pixelintensitätsdaten;
Vergleichen (S345) der gemittelten Pixelintensitätsdaten mit den angepassten gemittelten Pixelintensitätsdaten und
Identifizieren einer maximalen absoluten Differenz oder mehrerer maximaler absoluter Differenzen zwischen den gemittelten Pixelintensitätsdaten und den angepassten gemittelten Pixelintensitätsdaten.

6. Verfahren nach einem der vorhergehenden Ansprüche, ferner umfassend:
Identifizieren einer Pleuralinie im Ultraschallbild; und wobei sich die möglichen Bereiche von Interesse im Ultraschallbild von der Pleuralinie erstrecken.

7. Verfahren nach einem der vorhergehenden Ansprüche, ferner umfassend:
Erzeugen (S390, S399) von Anzeigedaten für das Ultraschallbild und Markierungen für mindestens eine von A-Linien-Strukturen oder B-Linien-Strukturen.

8. Verfahren nach einem der vorhergehenden Ansprüche, wobei der Empfangsschritt ein Empfangen einer Vielzahl von Ultraschallbildern, die eine Bildsequenz (Cineloop) bilden, umfasst und das Verfahren ferner umfasst:
Erzeugen einer zweiten aggregierten Bewertung für Nachhalle, wobei die zweite aggregierte Bewertung auf der Nachhallmerkmalsbewertung und der aggregierten Bewertung für Nachhalle aus jedem einer Teilmenge der Ultraschallbilder in der Bildsequenz basiert.

9. Ultraschallsystem (100), umfassend:
einen Prozessor (152, 252), der dazu ausgelegt ist, das Verfahren nach einem der Ansprüche 1 bis 8 auszuführen.

10. Ultraschallsystem nach Anspruch 9, ferner umfassend:
eine Anzeige (180) in Kommunikation mit dem Prozessor, wobei die Anzeige dazu ausgelegt ist, das Ultraschallbild und die Markierungen für mindestens eines von A-Linien-Strukturen oder B-Linien-Strukturen anzuzeigen.

11. Ultraschallsystem nach Anspruch 9 oder 10, ferner umfassend:
ein Ultraschallwandlerarray (113, 213) in Kommunikation mit dem Prozessor und dazu ausgelegt, das Ultraschallbild zu erfassen.

12. Computerprogrammprodukt, das Anweisungen umfasst, die bei Ausführung durch einen Prozessor bewirken, dass der Prozessor das Verfahren nach einem der Ansprüche 1 bis 8 ausführt.

## Revendications

1. Procédé mis en œuvre par ordinateur pour imagerie ultrasonore, le procédé comprenant : la réception (S315) d'une image ultrasonore à l'aide d'un réseau de transducteurs (113) d'une sonde ultrasonore (110) dans une procédure ultrasonore ;
la sélection (S330) de régions d'intérêt candidates (581B, 681B) dans l'image ultrasonore,
dans lequel chaque région d'intérêt candidate comprend une région essentiellement rectangulaire,
dans lequel un axe majeur de chaque région essentiellement rectangulaire est essentiellement perpendiculaire aux lignes A potentielles et/ou parallèle aux lignes B potentielles et dans lequel un axe mineur de chaque région rectangulaire est essentiellement parallèle aux lignes A potentielles et/ou perpendiculaire aux lignes B potentielles,
dans lequel les régions essentiellement rectangulaires ne se chevauchent essentiellement pas entre elles, et
dans lequel les régions essentiellement rectangulaires sont essentiellement parallèles entre elles ; et
la génération (S350), pour chaque région d'intérêt candidate, d'un score de caractéristiques de réverbérations,
la génération (S350), sur la base du score de caractéristiques de réverbérations pour chaque région candidate, d'un score agrégé pour les réverbérations dans l'image ultrasonore ;
l'estimation (S355), sur la base du score de caractéristiques de réverbérations et/ou du score agrégé pour les réverbérations, de la présence d'une ou de plusieurs structures de ligne A dans l'image ultrasonore ; et
l'estimation (S360), sur la base du score de caractéristiques de réverbérations et/ou du score agrégé pour les réverbérations, de la force d'une ou de plusieurs structures de ligne A dans l'image ultrasonore.

2. Procédé selon la revendication 1, comprenant en outre :
la classification (S370) d'une ou de plusieurs structures de ligne B dans l'image ultrasonore sur la base du score de caractéristiques de réverbérations et/ou du score agrégé pour les réverbérations.

3. Procédé selon la revendication 1 ou la revendication 2, comprenant en outre :
la génération (S320) de données de pixels pour l'image ultrasonore ; et
la détermination (S340) d'une intensité de pixels pour les données de pixels ; et
dans lequel le score de caractéristiques de réverbérations pour chaque région d'intérêt candidate est basé sur l'intensité de pixels à l'intérieur de ladite région d'intérêt candidate.

4. Procédé selon la revendication 3, dans lequel la sélection de régions d'intérêt candidates dans l'image ultrasonore comprend :
l'identification, sur la base de l'intensité de pixels dans l'image ultrasonore, d'un ou de plusieurs candidats de ligne B dans l'image ultrasonore ; et
la définition d'une région d'intérêt candidate renfermant chaque candidat de ligne B identifié.

5. Procédé selon la revendication 3 ou la revendication 4, dans lequel la génération du score de caractéristiques de réverbérations pour chaque région d'intérêt candidate sur la base de l'intensité de pixels à l'intérieur de ladite région d'intérêt candidate comprend pour chaque région d'intérêt :
le moyennage des données d'intensité de pixels ;
l'ajustement des données d'intensité de pixels moyennées ;
la comparaison (S345) des données d'intensité de pixels moyennées avec les données d'intensité de pixels moyennées ajustées ; et
l'identification d'une ou de plusieurs différences absolues maximales entre les données d'intensité de pixels moyennées et les données d'intensité de pixels moyennées ajustées.

6. Procédé selon l'une quelconque des revendications précédentes comprenant en outre :
l'identification d'une ligne pleurale dans l'image ultrasonore ; et
dans lequel les régions d'intérêt candidates dans l'image ultrasonore s'étendent à partir de la ligne pleurale.

7. Procédé selon l'une quelconque des revendications précédentes, comprenant en outre :
la génération (S390, S399) de données d'affichage pour l'image ultrasonore et de marqueurs pour au moins une des structures de ligne A ou des structures de ligne B.

8. Procédé selon l'une quelconque des revendications précédentes, dans lequel l'étape de réception comprend la réception d'une pluralité d'images ultrasonores formant une boucle cineloop, et le procédé comprend en outre :
la génération d'un second score agrégé pour les réverbérations, le second score agrégé étant basé sur le score de caractéristiques de réverbérations et le score agrégé pour les réverbérations à partir de chaque image ultrasonore d'un sous-ensemble des images ultrasonores dans la boucle cineloop.

9. Système à ultrasons (100), comprenant :
un processeur (152, 252) configuré pour réaliser le procédé selon l'une quelconque des revendications 1 à 8.

10. Système à ultrasons selon la revendication 9, comprenant en outre :
un écran (180) en communication avec le processeur, l'écran étant configuré pour afficher l'image ultrasonore et les marqueurs pour au moins une des structures de ligne A ou des structures de ligne B.

11. Système à ultrasons selon la revendication 9 ou la revendication 10 comprenant en outre :
un réseau de transducteurs ultrasonores (113, 213) en communication avec le processeur et configuré pour acquérir l'image ultrasonore.

12. Produit de programme informatique comprenant des instructions qui, lorsqu'elles sont exécutées par un processeur, amènent le processeur à réaliser le procédé selon l'une quelconque des revendications 1 à 8.
